**Europäisches Patentamt**

**European Patent Office**

**Office européen des brevets**

(19)

(11) Numéro de publication : **0 214 317 B1**

## (12) FASCICULE DE BREVET EUROPÉEN

(45) Date de publication du fascicule du brevet :
17.08.88

(51) Int. Cl.⁴ : **A 23 L   1/308**

(21) Numéro de dépôt : **85110944.7**

(22) Date de dépôt : **30.08.85**

(54) Produit diététique à activité dépurative et anti-diarrhéique et son procédé de préparation.

(43) Date de publication de la demande :
18.03.87 Bulletin 87/12

(45) Mention de la délivrance du brevet :
17.08.88 Bulletin 88/33

(84) Etats contractants désignés :
**AT BE CH DE FR GB IT LI NL SE**

(56) Documents cités :
**FR-A- 2 233 946**
**US-A- 4 379 782**

(73) Titulaire : **SOCIETE DES PRODUITS NESTLE S.A.**
**Case postale 353**
**CH-1800 Vevey (CH)**

(72) Inventeur : **Würsch, Pierre**
**Ch. de la Crausaz 64**
**CH-1814 La Tour-de-Peilz (CH)**

## Description

La présente invention concerne un produit diététique à activité dépurative et anti-diarrhéique.

Les maladies diarrhéiques, très répandues, constituent un facteur très important de la mortalité infantile et même adulte. La diarrhée consiste en une émission trop fréquente et trop précipitée de selles mal formées et est caractérisée par l'élimination d'un excès d'eau dans les selles conduisant progressivement à une déshydratation. Ce phénomène paraît provoqué par une malabsorption, une sécrétion excessive ou encore un défaut de la motricité intestinale, ces défauts pouvant être concomitants. Bien que les causes en soient multiples, il est admis que les diarrhées ont pour origine principale la présence de toxines bactériennes ou virales dans le tractus intestinal.

En simplifiant, on traite actuellement les diarrhées par des produits absorbant l'eau, les toxines, virus ou bactéries selon un phénomène physique ou par des médicaments stimulant l'absorption, inhibant la sécrétion ou modifiant la motricité intestinale ou encore antibiotiques ou à l'aide de solutions de réhydratation contenant de l'eau et des sels minéraux et administrées par voie orale ou intra-veineuse.

Les médicaments ont l'inconvénient de ne pas être toujours efficaces et d'avoir des effets secondaires. Les produits absorbant l'eau, par exemple les gommes, les pectines, la méthylcellulose, la carotte, peuvent modifier la fréquence et la consistance des selles mais ne diminuent en aucune façon les pertes d'eau et d'électrolytes. Les solutions de réhydratation permettent de compenser ces pertes mais atténuent les effets des diarrhées au lieu d'agir sur leurs causes. Les produits capables d'absorber les toxines, virus et bactéries, par exemple le carbonate de calcium, le silicate d'aluminium hydraté, les sels de bismuth, les pectines, le charbon activé sont très populaires, mais il n'est pas certain qu'ils soient véritablement efficaces. Les résines échangeuses d'ions, qui ont en plus la propriété de fixer les sels biliaires semblent efficaces mais ont également des effets secondaires et doivent être employées à des doses élevées.

Il existe sur le marché une composition diététique contenant 60 à 80 % de farine de caroube torréfiée (AROBON®). Si ce produit donne de bons résultats dans le traitement des diarrhées, sans présenter d'effets secondaires, il a l'inconvénient de nécessiter des doses relativement élevées, de l'ordre de 20-40 g/jour pour les jeunes enfants, ce qui pose des problèmes d'administration.

L'invention met à disposition un produit diététique actif dans le traitement des diarrhées ne présentant pas les inconvénients mentionnés ci-dessus.

Le produit diététique selon l'invention est caractérisé par le fait qu'il comprend comme principe actif une farine de caroube contenant au moins 20 % en poids des matières sèches de tanins natifs exprimés en polyphénols totaux et dont le rapport pondéral tanins solubles/tanins insolubles est inférieur à 0,37, la solubilité étant déterminée dans l'eau à 37 °C.

On entend par tanins natifs selon l'invention que ceux-ci n'ont pas subi de dénaturation à la suite d'un traitement thermique ou chimique. Par exemple, lorsque la gousse de caroube (Ceratonia siliqua) non ou partiellement désucrée subit une torréfaction, il se produit une maillardisation qui détruit la structure des tanins, puisqu'on ne retrouve plus par examen microscopique les cristaux caractéristiques des tanins condensés de caroube. De même, si par exemple on traite la caroube par des acides, les tanins se transforment en phlobaphènes par polymérisation.

Dans la caroube mûre, la plupart des tanins présents sont sous forme condensée, c'est-à-dire de polymères formés de sous-unités de flavanol-3 et de leurs esters galliques. Ces tanins sont insolubles dans l'eau froide ou tiède et en particulier à la température du corps, à 37 °C. Ceci a pour conséquence qu'ils parviennent dans l'intestin sans être dégradés par l'acide gastrique ou inactivés par les protéines. Ils peuvent donc jouer leur rôle dépuratif et antiseptique sans intervenir dans les processus physiologiques.

L'évaluation de la teneur peut être faite en dosant la totalité des polyphénols, par exemple en appliquant la méthode colorimétrique de Folin-Denis, « Tannin » dans Official Methods of Analysis (1980), W. Horwitz, ed., Association of Official Analytical Chemists, Washington D.C. selon une procédure modifiée. Cette modification consiste à doser d'abord les tanins solubles, extraits à l'eau à 37 °C sous agitation modérée pendant 15 min., dans le surnageant obtenu après décantation. On dose ensuite les tanins condensés, qui sont insolubles dans l'eau à 37 °C, qu'on extrait de la phase solide par le diméthylformamide à 120 °C pendant 4 h avec agitation intermittente toutes les 20 min. On peut aussi doser les tanins par gravimétrie selon L. Vuataz, H. Brandenberger et R. Egli, J. Chromatogr. (1959), vol. 2, p. 173 à 187.

De préférence, la farine de caroube est enrichie en tanins par rapport à la gousse de caroube mûre. Elle contient avantageusement 30 à 90 % de tanins exprimés en polyphénols totaux en poids des matières sèches. Parmi ces tanins, les solubles représentent avantageusement moins de 10 %, de préférence moins de 6 %, par exemple environ 4 % en poids. Le rapport pondéral tanins solubles/tanins insolubles est ainsi de préférence 0,04 à 0,1.

Le produit diététique selon l'invention peut se présenter sous toute forme adaptée à l'administration orale. Cela peut être une poudre à disperser dans un milieu aqueux, par exemple l'eau, le lait, un jus de fruits, une boisson isotonique. Avantageusement, une telle poudre contiendra en plus de la farine de caroube des additifs de qualité alimentaire, par exemple des agents effervescents, par exemple un

**0 214 317**

mélange d'acide et de carbonate, des agents mouillants, par exemple la lécithine, des arômes, des sels de réhydratation, des peptides provenant par exemple de protéines hydrolysées. Elle peut également contenir des sucres, avantageusement de la maltodextrine, du dextrose ou de préférence du saccharose, par exemple en quantité nécessaire pour standardiser le pourcentage pondéral de tanins, exprimés en polyphénols totaux, de la préparation à une valeur prédéterminée, de préférence environ 22 % en poids.

En variante, le produit diététique peut se présenter sous forme de granulés, comprimés ou dragées à croquer ou à dissoudre, le cas échéant efferverscents, préparés à partir de la farine par les méthodes habituelles.

Enfin, le produit diététique peut consister en un aliment, avantageusement un dessert, par exemple une crème, une gelée ou un flan dans lequel est incorporée la farine de caroube.

La quantité administrée dépendra bien entendu de l'âge du patient (nourrisson, enfant ou adulte), de la nature et de la gravité de la diarrhée à traiter, ainsi que de la nature du traitement lui-même (préventif ou curatif). A titre indicatif, une dose comprenant de 1 à 2 g de farine de caroube/kg de poids corporel et par jour de préférence 1 g/kg/jour jusqu'à 10 kg de poids corporel et jusqu'à 20 g/jour au-delà de 10 kg de poids corporel permet un traitement de la diarrhée en 8 à 10 jours. La farine pourra être dispersée dans un milieu aqueux, à raison de 1 à 3 % en poids.

La farine peut être emballée de préférence sous atmosphère inerte, par exemple, en boîtes de 100 g avec mesurette ou en sachets doses de 5-10 g étanches à l'air, à la lumière et à l'humidité.

La farine de caroube ci-dessus a une activité bactéricide sur Staphylococcus aureus et E. Coli entéropathogène et bactériostatique sur E. Coli. L'effet inhibiteur est probablement dû à l'adsorption mécanique des bactéries sur les granules de tanins solubles à partir de ces granules. On a également observé que ces granules de tanins adsorbaient l'entérotoxine du choléra et l'entérotoxine thermolabile de E. Coli et qu'ils inactivaient les entérovirus. Aux doses ingérées ces tanins ne perturbent pas le processus normal de la digestion en dépit de leur activité antinutritionnelle connue par inhibition des enzymes digestives. Ils ont une action de détoxification, un effet retard et ne sont pas absorbés contrairement aux antibiotiques habituellement prescrits.

L'invention concerne également un procédé de préparation de la farine de caroube, caractérisé par le fait qu'on traite des gousses de caroube mûres par un milieu aqueux de manière à en extraire la plus grande partie des sucres et des tanins hydrosolubles, qu'on sépare le résidu, qu'on le sèche à une température ne dépassant pas 100 °C et qu'on le moud en particules de diamètre inférieur ou égal à 200 µm.

Les gousses de caroube mûres servant de matière première se caractérisent par un pourcentage de sucres ayant atteint le maximum du fait de la maturation, soit environ 50 % en poids dont environ 40 % en poids de saccharose et une humidité inférieure à 20 % en poids. On peut de préférence les concasser en morceaux de diamètre inférieur à 1,5 cm, le cas échéant après les avoir congelés. La caroube sans les graines a ainsi la composition type suivante :

| | % pondéral de matières sèches |
|---|---|
| Glucides solubles | 49 |
| Polyphénols solubles | 1,2 |
| Tanins insolubles (déterminés par gravimétrie) | 19,8 |
| dont polyphénols | (11) |
| Pectine, hemicellulose-lignocellulose | 24,5 |
| Protéines (N × 6,25) | 3 |
| Cendres | 2,5 |

Selon un premier mode de réalisation du procédé en continu, qui est préféré, on moud la caroube en présence d'eau à froid, par exemple à 15-20 °C, par exemple dans un moulin colloïdal. La quantité d'eau ajoutée est de préférence 5 à 15 fois la quantité de caroube. Après séparation de la phase liquide, par exemple dans un décanteur, on recueille la caroube désucrée.

De préférence, on traite thermiquement la caroube désucrée, par exemple en y ajoutant un poids équivalent d'eau chaude, puis on pasteurise la dispersion, par exemple par injection directe de vapeur à 95-98 °C pendant 30 s à 10 min. Ce traitement thermique a l'avantage d'éliminer de la dispersion la majeure partie des microorganismes avant d'effectuer sa mouture ultérieure.

On moud ensuite la dispersion pasteurisée, de préférence d'abord dans un moulin à dents, puis dans un moulin à meules à une température ne dépassant pas environ 90 °C.

On sèche ensuite la dispersion, de préférence par pulvérisation dans une tour. Le séchage a lieu dans des conditions permettant d'éviter la dénaturation thermique des tanins, c'est-à-dire à une température ne dépassant pas 100 °C et de préférence de 90 °C au niveau du produit.

On moud enfin la poudre obtenue à sec, par exemple dans un moulin à broches jusqu'à obtenir une farine dont les particules ont un diamètre inférieur ou égal à 200 µm, avantageusement de 10 à 200 µm, et de préférence environ 100 µm, ce qui permet de ne pas déceler de granulosité désagréable dans le produit diététique contenant la farine et d'éviter la sédimentation rapide des particules, par exemple dans une boisson.

Selon un second mode de réalisation, par charges, on met la caroube concassée, de préférence en

3

particules de diamètre 0,8 à 5 mm, en présence d'eau chaude, de préférence à 60-98 °C. On peut remplir des colonnes avec la caroube concassée et les faire traverser par de l'eau ou en variante mettre la caroube concassée en suspension dans de l'eau dans des cuves et agiter la suspension. On recueille ensuite la phase solide, par exemple par gravité ou par décantation, qu'on sèche à l'air chaud. On peut par exemple disposer le résidu humide dans des plateaux dans un sécheur dans lequel règne, de préférence un vide partiel et une température de 60 à 70 °C pendant 24 à 72 h. On moud enfin la matière séchée, par exemple dans un moulin à broches, jusqu'à obtenir des particules de diamètre 10 à 200 μm et de préférence environ 10 μm.

Selon une variante des modes de réalisation précédents permettant d'enrichir la farine en tanins, on peut sécher la dispersion ou le résidu, par exemple par lyophilisation, le transformer en granules, par exemple au moyen d'une assiette à granuler puis écraser les granules, par exemple à l'aide d'un moulin à meules ou à cylindres et éliminer la majeure partie des fines, par exemple par élutriation dans un courant d'air chaud.

Les particules obtenues ont de préférence des diamètres de 10 à 200 μm.

Les exemples suivants illustrent l'invention. Dans ceux-ci, les pourcentages sont pondéraux, sauf mention contraire.

## Exemple 1

Dans un moulin colloïdal, on fait passer en continu 1,7 kg/min de gousses de caroube mûres égrainées, concassées en particules de diamètre moyen 2 cm, ayant 95 % de matières sèches, en présence de 21 l/min d'eau courante à 18 °C, la dispersion étant évacuée à travers une grille dont les orifices ont 1 mm. Après un temps d'attente de 6 min, on fait passer la dispersion dans un décanteur dans lequel on sépare la phase liquide et on recueille la phase solide contenant 18 % de fibres, exprimés en matières sèches.

Après un mélange de la phase solide avec d'autant d'eau courante à 95 °C, on pasteurise la dispersion par injection directe de vapeur à 95-97 °C pendant 30 s, puis on la moud à 85 °C d'abord dans un moulin à dents, puis dans un moulin à meules. On sèche ensuite la dispersion moulue par pulvérisation dans une tour à un débit de 175 l/h, la température de l'air d'entrée étant 165-170 °C et celle de l'air de sortie 96-98 °C. Dans ces conditions, la température au niveau du produit ne dépasse pas 90 °C. On moud enfin la poudre obtenue à sec dans un moulin à broches tournant à 9 000 t/min.

Les particules obtenues ont 3 % de matières sèches. 50 % de celles-ci passent à travers un tamis dont les mailles ont 75 μm et 100 % à travers un tamis à mailles de 200 μm.

La farine a la composition suivante exprimée en matières sèches :

|  | % |
|---|---|
| Glucides solubles | 19,3 |
| Tanins (gravimétrie) | 47,4 |
| dont polyphénols | (27) |
| Fibres | 25,7 |
| Protéines | 4,8 |
| Cendres | 2,8 |

Le rapport des tanins solubles et insolubles est 0,23 : 1.

## Exemple 2

On congèle à — 40 °C 127 kg de gousses de caroube mûres égrainées concassées contenant 89 % de matières sèches, on les découpe dans un trancheur avec une ouverture de 3 mm, puis on élimine les fines à travers un tamis dont les mailles ont 0,8 mm. On dispose les 108 kg restant dans des colonnes. Après avoir fait gonfler les particules dans de l'eau désionisée, on fait passer de l'eau désionisée à 70 °C à travers les colonnes avec un débit de 180 l/h. Après drainage des colonnes par gravité on recueille 210 kg de particules solides contenant 19 % de matières sèches. On dispose les particules humides sur des plateaux dans un sécheur à air et on les sèche pendant 48 h à la température de l'air de 80 °C et à une pression de 60 mb. On obtient ainsi 42,5 kg de matière séchée contenant 89,5 % de matières sèches. On moud enfin cette manière dans un moulin multi-broches tournant à 12.000 t/min avec un débit de 40 kg/h, ce qui conduit à 40 kg de farine dont les particules ont une teneur en matières sèches de 92 % et un diamètre moyen de 100 μm.

La farine a la composition suivante exprimée en matières sèches :

|  | % |
|---|---|
| Glucides solubles | 22,5 |
| Tanins (gravimétrie) | 36,2 |
| dont polyphénols | (22,6) |
| Fibres | 34,9 |

4

| | |
|---|---|
| Protéines | 4,6 |
| Cendres | 1,8 |

Le rapport des tanins solubles et insolubles est 0,06 : 1.

## Exemple 3

On traite 29 kg de gousses de caroube mûres ayant été entreposées pendant au moins 6 mois, égrainées, concassées et moulues en particules de diamètre moyen 5 mm par 100 kg d'eau désionisée à 98 °C pendant 10 min dans une cuve sous agitation. On essore la dispersion et on recueille 34,5 kg de résidu qu'on lave à 15 °C avec 100 kg d'eau désionisée pendant 10 min. Après un nouvel essorage de la dispersion, on recueille 27,2 kg de résidu qu'on sèche par lyophilisation. Après granulation en particules de diamètre moyen 3 mm sur une assiette à granuler, on écrase les particules dans un moulin à cylindres et on élimine les fibres par un courant d'air chaud. Une mouture finale avec un moulin microniseur conduit à une farine enrichie en tanins insolubles par rapport à la caroube de départ et dont les particules ont un diamètre moyen de 25 μm.

La farine a la composition suivante, exprimée en matières sèches :

| | % |
|---|---|
| Glucides solubles | — |
| Tanins (gravimétrie) | 71,7 |
| dont polyphénols | (41,9) |
| Fibres | 22,6 |
| Protéines et cendres | 5,7 |

Le rapport des tanins solubles et insolubles est 0,04 : 1.

## Exemple 4

On mélange à sec 82 % de farine de caroube préparée selon l'exemple 1 avec 17,9 % de saccharose finement moulu contenant 3 % de lécithine de soja et 0,1 % de vanilline pendant 30 min., puis on moud le mélange dans un moulin muni d'une grille dont les trous ont 0,8 mm. On emballe ensuite la poudre dans des boîtes de 160 g étanches à la lumière, à l'air et à l'humidité munies d'une mesurette.

## Exemple 5

Granulés effervescents

Prémélange I

On mélange à sec 80 g de gélatine, 2 kg de farine de caroube préparée selon l'exemple 1 et 160 g de maltodextrine d'équivalent dextrose 5 %.

Dans un pétrin, on ajoute au mélange précédent 4 l d'eau froide désionisée et on mélange le tout de manière homogène. On sèche ensuite la pâte dans un sécheur à plateaux à 40-50 °C pendant 12 h.

Prémélange II

On prépare un prémélange effervescent contenant 25 % d'acide citrique, 52,3 % de bicarbonate de sodium et 22,7 % d'acide tartrique, puis on ajoute à 75,2 g de ce prémélange effervescent 21,6 g de cire végétale en poudre, 4,8 g de stéarate de magnésium et 50,4 g d'arôme orange.

On mélange intimement les prémélanges I et II puis on les moud en granulés de diamètre moyen 1,5 mm.

## Exemple 6

Comprimés effervescents

A partir des granulés obtenus selon l'exemple 5, on prépare des comprimés effervescents de 0,65 g, de diamètre 1 cm et d'épaisseur 7,2 mm en comprimant ces granulés dans une chambre cylindrique de 1 cm de diamètre et de 16 mm de profondeur.

## Exemple 7

Comprimés à croquer

On prépare des comprimés à croquer à partir des ingrédients ci-après :

|  | % |
|---|---|
| Farine de caroube selon l'exemple 1 | 81,4 |
| Protéine de maïs | 7,1 |
| Graisse alimentaire | 3,4 |
| Arôme menthe | 1,5 |
| Maltodextrine | 6,2 |
| Stéarate de calcium | 0,4 |

On mélange les différents ingrédients à sec et on les comprime comme à l'exemple 6.

Exemple 8

Granulés à croquer

Après un mélange à sec des ingrédients ci-après :

|  | % |
|---|---|
| Farine de caroube selon l'exemple 1 | 80 |
| Extrait de malt | 10 |
| Beurre de cacao | 10 |

On confectionne des granulés à l'aide d'un compacteur à roues dentées creuses munies de perforations de diamètre 2-3 mm. Des racleurs placés à l'intérieur des roues découpent les boudins sortant des perforations en granulés qui sont ensuite enrobés d'un film de gomme arabique et giclés avec un sirop de sucre aromatisé.

Exemple 9

Lait aromatisé

On prépare un lait aromatisé en mélangeant 15 g de poudre de lait entier contenant 50 % de saccharose avec 6 g de farine obtenue selon l'exemple 1 et des traces d'arôme de fraise.
On obtient une boisson par dilution de ce mélange dans 100 ml d'eau.

Exemple 10

Crème

On confectionne une crème au chocolat à partir de 50 g de mélange en poudre pour crème au chocolat auquel on ajoute 12 g de farine de caroube selon l'exemple 1 et on dilue le tout dans 100 ml de lait froid. La crème obtenue est homogène et la présence de la farine de caroube n'est pas perceptible.

Exemple 11

Poudre pour réhydratation orale

On mélange à sec les ingrédients ci-après dans les quantités indiquées :

| Chlorure de potassium | 1,5 g |
|---|---|
| Chlorure de sodium | 3,5 g |
| Hydrogénocarbonate de sodium | 2,5 g |
| Glucose monohydrate | 20 g |
| Farine selon l'exemple 1 | 10-15 g |

Au moment de l'emploi, on dilue ce mélange dans 1 l d'eau courante, de préférence stérilisée au préalable. Le volume de liquide à ingérer pour les enfants est 50-100 ml/kg de poids corporel/4 h et jusqu'à 750 ml/h pour les adultes, la quantité de farine ingérée par jour ne dépassant pas 20 g.

Exemple 12

On a étudié l'effet de la farine obtenue selon l'exemple 1 sur la diarrhée aiguë (6-12 selles/jour) des enfants causée en prédominance par E. Coli. 33 enfants de 5 à 30 mois ont reçu 1 g de farine de caroube par kg de poids corporel, mélangée à du porridge. Dans 28 cas (85 %), la diarrhée a été stoppée en 48-72 h.

# 0 214 317

On a administré à un autre groupe de 31 enfants de 1-12 mois souffrant de diarrhée aiguë (plus de 5 selles/jour) 5 g par kg de poids corporel et au maximum 40 g/jour de farine de caroube torréfiée du commerce (AROBON®) dans un lait à raison de 5 %. 26 cas (84 %) ont été guéris en 7 jours.

Un autre groupe comparatif, traité par un antibiotique, le chloramphénicol, montrait 64 % de guérison au bout de 7 jours.

On voit donc que la farine de carboube préparée selon l'invention est plus active à une dose 5 fois moindre que la farine de caroube torréfiée et qu'un antibiotique couramment prescrit tout en ne présentant pas les inconvénients de ce dernier (effets secondaires, détérioration de la flore intestinale).

**Revendications** (pour les Etats contractants : BE, CH, DE, FR, GB, IT, LI, NL, SE)

1. Produit diététique à activité dépurative et anti-diarrhéique, caractérisé par le fait qu'il comprend comme principe actif une farine de caroube contenant au moins 20 % en poids des matières sèches de tanins natifs exprimés en polyphénols totaux et dont rapport pondéral tanins solubles/tanins insolubles est inférieur à 0,37, la solubilité étant déterminée dans l'eau à 37 °C.

2. Produit selon la revendication 1, caractérisé par le fait que la farine de caroube contient 30 à 90 % en poids de tanins natifs exprimés en polyphénols totaux.

3. Produit selon la revendication 1, caractérisé par le fait qu'il se présente sous une forme adaptée à l'administration orale.

4. Produit selon la revendication 3, caractérisé par le fait qu'il contient des sels de réhydratation.

5. Procédé de préparation de la farine de caroube entrant dans la composition du produit selon la revendication 1, caractérisé par le fait qu'on traite des gousses de caroube mûres par un milieu aqueux de manière à en extraire la plus grande partie des sucres et des tanins hydrosolubles, qu'on sépare le résidu, qu'on le sèche à une température ne dépassant pas 100 °C et qu'on le moud en particules de diamètre inférieur ou égal à 200 µm.

6. Procédé selon la revendication 5, caractérisé par le fait qu'on procède en continu, qu'on moud la caroube en présence d'eau à froid, qu'on sépare la phase liquide, qu'on recueille le résidu, qu'on le pasteurise en présence d'eau courante à 95-98 °C pendant 30 s à 10 mn, qu'on moud finement la dispersion pasteurisée, qu'on la sèche par pulvérisation et qu'on moud la dispersion séchée en particules de diamètre inférieur ou égal à 200 µm.

7. Procédé selon la revendication 5, caractérisé par le fait qu'on procède par charges, qu'on moud la caroube à sec en particules de diamètre 0,8 à 5 mm, qu'on met les particules en présence d'eau à une température de 60 à 98 °C, qu'on recueille la phase solide, qu'on la sèche dans un sécheur à air le cas échéant à une pression sub-atmosphérique à une température de l'air inférieure à 80 °C et qu'on moud le résidu en particules de diamètre inférieur ou égal à 200 µm.

8. Procédé selon la revendication 5, caractérisé par le fait qu'on sèche le résidu par lyophilisation, qu'on le transforme en granules, qu'on écrase les granules et qu'on élimine la majeure partie des fines par un courant d'air chaud.

9. Procédé de préparation d'un produit diététique selon la revendication 3, caractérisé par le fait qu'on mélange la farine de caroube avec des additifs alimentaires par voie humide ou sèche et qu'on transforme le cas échéant le mélange en granulés ou en comprimés.

10. Procédé de préparation d'un produit diététique selon la revendication 3, caractérisé par le fait qu'on incorpore la farine de caroube dans une boisson ou un dessert.

**Revendications** (pour l'Etat contractant AT)

1. Procédé de préparation d'une farine de caroube contenant au moins 20 % en poids des matières sèches de tanins natifs exprimés en polyphénols totaux et dont rapport pondéral tanins solubles/tanins insolubles est inférieur à 0,37, la solubilité étant déterminée dans l'eau à 37 °C, caractérisé par le fait qu'on traite des gousses de caroube mûres par un milieu aqueux de manière à en extraire la plus grande partie des sucres et des tanins hydrosolubles, qu'on sépare le résidu, qu'on le sèche à une température ne dépassant pas 100 °C et qu'on le moud en particules de diamètre inférieur ou égal à 200 µm.

2. Procédé selon la revendication 1, caractérisé par le fait qu'on procède en continu, qu'on moud la caroube en présence d'eau à froid, qu'on sépare la phase liquide, qu'on recueille le résidu, qu'on le pasteurise en présence d'eau courante à 95-98 °C pendant 30 s à 10 mn, qu'on moud finement la dispersion pasteurisée, qu'on la sèche par pulvérisation et qu'on moud la dispersion séchée en particules de diamètre inférieur ou égal à 200 µm.

3. Procédé selon la revendication 1, caractérisé par le fait qu'on procède par charges, qu'on moud la caroube à sec en particules de diamètre 0,8 à 5 mm, qu'on met les particules en présence d'eau à une température de 60 à 98 °C, qu'on recueille la phase solide, qu'on la sèche dans un sécheur à air le cas échéant à une pression subatmosphérique à une température de l'air inférieure à 80 °C et qu'on moud le résidu en particules de diamètre inférieur ou égal à 200 µm.

4. Procédé selon la revendication 1, caractérisé par le fait qu'on sèche le résidu par lyophilisation, qu'on le transforme en granules, qu'on écrase les granules et qu'on élimine la majeure partie des fines par un courant d'air chaud.

5. Procédé selon la revendication 1, caractérisé par le fait que la farine de caroube contient 30 à 90 % en poids de tanins natifs exprimés en polyphénols totaux.

6. Procédé de préparation d'un produit diététique sous une forme adaptée à l'administration orale, caractérisé par le fait qu'on mélange la farine de caroube avec des additifs alimentaires par voie humide ou sèche et qu'on transforme le cas échéant le mélange en granulés ou en comprimés.

7. Procédé de préparation d'un produit diététique sous une forme adaptée à l'administration orale, caractérisé par le fait qu'on incorpore la farine de caroube dans une boisson ou un dessert.

8. Procédé selon l'une des revendications 6 et 7, caractérisé par le fait qu'on incorpore au produit diététique des sels de réhydratation.

**Claims** (for the Contracting States : BE, CH, DE, FR, GB, IT, LI, NL, SE)

1. A dietetic product with depurative and antidiarrhoeic activity, characterized in that it comprises as active principle a carob flour containing at least 20 % by weight, based on dry matter, of native tannins expressed as total polyphenols, of which the ratio by weight of soluble tannins to insoluble tannins is below 0.37, solubility being determined in water at 37 °C.

2. A product as claimed in Claim 1, characterized in that the carob flour contains from 30 to 90 % by weight of native tannins expressed as total polyphenols.

3. A product as claimed in Claim 1, characterized in that it is presented in a form suitable for oral administration.

4. A product as claimed in Claim 3, characterized in that it contains rehydratation salts.

5. A process for preparing the carob flour entering into the composition of the product claimed in Claim 1, characterized in that ripe carob pods are treated with an aqueous medium to extract most of the sugars and watersoluble tannins, in that the residue is separated and dried at a temperature not exceeding 100 °C and in that it is ground into particles 200 μm or less in diameter.

6. A process as claimed in Claim 5, characterized in that it is carried out continuously, in that the carob is ground in the presence of cold water, in that the liquid phase is separated, in that the residue is collected, in that it is pasteurized in the presence of running water at 95-98 °C for 30 s to 10 mn, in that the pasteurized dispersion is finely ground, in that it is spray-dried and in that the dried dispersion is ground into particles 200 μm or less in diameter.

7. A process as claimed in Claim 5, characterized in that it is carried out in batches, in that the carob is dry-ground into particles 0,8-5 mm in diameter, in that the particles are introduced into water at 60-98 °C, in that the solid phase is collected, in that it is dried in an air dryer, optionally at a sub-atmospheric pressure, at an air temperature below 80 °C and in that the residue is ground into particles 200 μm or less in diameter.

8. A process as claimed in Claim 5, characterized in that the residue is dried by freeze drying, in that it is converted into granules, in that the granules are crushed and in that most of the fines are eliminated by a stream of hot air.

9. A process for preparing the dietetic product claimed in Claim 3, characterized in that the carob flour is wet- or dry-mixed with food additives and in that the mixture is optionally converted into granulates or tablets.

10. A process for preparing the dietetic product claimed in Claim 3, characterized in that the carob flour is incorporated in a beverage or dessert.

**Claims** (for the Contracting State AT)

1. A process for preparing a carob flour containing at least 20 % by weight, based on dry matter, of native tannins expressed as total polyphenols, of which the ratio by weight of soluble tannins to insoluble tannins is below 0.37, solubility being determined in water at 37 °C characterized in that ripe carob pods are treated with an aqueous medium to extract most of the sugars and water-soluble tannins, in that the residue is separated and dried at a temperature not exceeding 100 °C and in that it is ground into particles 200 μm or less in diameter.

2. A process as claimed in Claim 1, characterized in that it is carried out continuously, in that the carob is ground in the presence of cold water, in that the liquid phase is separated, in that the residue is collected, in that it is pasteurized in the presence of running water at 95-98 °C for 30 s to 10 mn, in that the pasteurized dispersion is finely ground, in that it is spray-dried and in that the dried dispersion is ground into particles 200 μm or less in diameter.

3. A process as claimed in Claim 1, characterized in that it is carried out in batches, in that the carob is dryground into particles 0.8-5 mm in diameter, in that the particles are introduced into water at 60-98 °C, in that the solid phase is collected, in that it is dried in an air dryer, optionally at a sub-atmospheric pressure, at an air temperature below 80 °C and in that the residue is ground into particles 200 μm or less

in diameter.

4. A process as claimed in Claim 1, characterized in that the residue is dried by freeze drying, in that it is converted into granules, in that the granules are crushed and in that most of the fines are eliminated by a stream of hot air.

5. A process as claimed in Claim 1, characterized in that the carob flour contains from 30 to 90 % by weight of native tannins expressed as total polyphenols.

6. A process preparing a dietetic product in a form suitable for oral administration, characterized in that the carob flour is wet- or dry-mixed with food additives and in that the mixture is optionally converted into granulates or tablets.

7. A process for preparing a dietetic product in a form suitable for oral administration characterized in that the carob flour is incorporated in a beverage or dessert.

8. A process as claimed in claim 6 or 7, characterized in that rehydratation salts are incorporated in the dietetic product.

**Patentansprüche** (für die Vertragsstaaten : BE, CH, DE, FR, GB, IT, LI, NL, SE)

1. Diätetisches Produkt mit depurativer und Anti-Diarrhöe-Wirkung, dadurch gekennzeichnet, daß es als Wirkstoff ein Johannisbrotmehl enthält, welches wenigstens 20 Gew.-%, bezogen auf die Trockensubstanz, an natürlichen, als Gesamtpolyphenole ausgedrückten Tanninen enthält, und bei welchem das Gewichtsverhältnis von löslichen Tanninen/unlöslichen Tanninen kleiner als 0,37 ist, wobei die Löslichkeit in Wasser von 37 °C bestimmt worden ist.

2. Produkt nach Anspruch 1, dadurch gekennzeichnet, daß das Johannisbrotmehl 30 bis 90 Gew.-% an natürlichen, als Gesamtpolyphenole ausgedrückten Tanninen enthält.

3. Produkt nach Anspruch 1, dadurch gekennzeichnet, daß es in einer für die orale Verabreichung geeigneten Form vorliegt.

4. Produkt nach Anspruch 3, dadurch gekennzeichnet, daß es Rehydratisierungssalze enthält.

5. Verfahren zur Herstellung von Johannisbrotmehl zur Verwendung in der Zusammensetzung für das Produkt nach Anspruch 1, dadurch gekennzeichnet, daß man reife Johannisbrotschoten mit einem wäßrigen Medium derart behandelt, daß aus den Schoten der größte Teil der Zucker und der wasserlöslichen Tannine extrahiert wird, daß man den Rückstand abstrennt, daß man denselben bei einer Temperatur, welche 100 °C nicht überschreitet, trocknet, und daß man denselben zu Teilchen mit einem Durchmesser von kleiner als oder gleich 200 μm vermahlt.

6. Verfahren nach Anspruch 5, dadurch gekennzeichnet, daß man kontinuierlich arbeitet, daß man das Johannisbrot in Gegenwart von kaltem Wasser vermahlt, daß man die flüssige Phase abtrennt, daß man den Rückstand sammelt, daß man denselben in Gegenwart von fließendem Wasser von 95 bis 98 °C während 30 sec bis 10 mn pasteurisiert, daß man die pasteurisierte Dispersion feinvermahlt, daß man dieselbe durch Zerstäubung trocknet, und daß man die getrocknete Dispersion zu Teilchen mit einem Durchmesser von kleiner als oder gleich 200 μm vermahlt.

7. Verfahren nach Anspruch 5, dadurch gekennzeichnet, daß man chargenweise arbeitet, daß man das Johannisbrot trocken zu Teilchen mit einem Durchmesser von 0,8 bis 5 mm vermahlt, daß man die Teilchen in Gegenwart von Wasser mit einer Temperatur von 60 bis 98 °C beläßt, daß man die feste Phase sammelt, daß man dieselbe in einem Lufttrockner und gegebenenfalls bei einem unter Atmosphärendruck liegenden Druck bei einer Lufttemperatur von unter 80 °C trocknet, und daß man den Rückstand zu Teilchen mit einem Durchmesser von kleiner als oder gleich 200 μm vermahlt.

8. Verfahren nach Anspruch 5, dadurch gekennzeichnet, daß man den Rückstand durch Lyophilisieren trocknet, daß man denselben granuliert, daß man die Körnchen zerstößt, und daß man den Hauptanteil der Feinteilchen durch einen Heißluftstrom entfernt.

9. Verfahren zur Herstellung eines diätetischen Produktes nach Anspruch 3, dadurch gekennzeichnet, daß man das Johannisbrotmehl auf naßem oder trockenem Wege mit Nahrungmittelzusätzen vermischt, und daß man die Mischung gegebenenfalls in ein Granulat oder zu Tabletten umwandelt.

10. Verfahren zur Herstellung eines diätetischen Produktes nach Anspruch 3, dadurch gekennzeichnet, daß man das Johannisbrotmehl einem Getränk oder einer Nachspeise einverleibt.

**Patentansprüche** (für den Vertragsstaat AT)

1. Verfahren zur Herstellung eines Johannisbrotmehls, welches wenigstens 20 Gew.-%, bezogen auf die Trockensubstanz, an natürlichen, als Gesamtpolyphenole ausgedrückten Tanninen enthält, und bei welchem das Gewichtsverhältnis von löslichen Tanninen/unlöslichen Tanninen kleiner als 0,37 ist, wobei die Löslichkeit in Wasser von 37 °C bestimmt worden ist, dadurch gekennzeichnet, daß man reife Johannisbrotschoten mit einem wäßrigen Medium derart behandelt, daß aus den Schoten der größte Teil der Zucker und der wasserlöslichen Tannine extrahiert wird, daß man den Rückstand abstrennt, daß man denselben bei einer Temperatur, welche 100 °C nicht überschreitet, trocknet, und daß man denselben zu Teilchen mit einem Durchmesser von kleiner als oder gleich 200 μm vermahlt.

2. Verfahren nach Anspruch 1, dadurch gekennzeichnet, daß man kontinuierlich arbeitet, daß man das Johannisbrot in Gegenwart von kaltem Wasser vermahlt, daß man die flüssige Phase abtrennt, daß man den Rückstand sammelt, daß man denselben in Gegenwart von fließendem Wasser von 95 bis 98 °C während 30 sec bis 10 min pasteurisiert, daß man die pasteurisierte Dispersion feinvermahlt, daß man dieselbe durch Zerstäubung trocknet, und daß man die getrocknete Dispersion zu Teilchen mit einem Durchmesser von kleiner als oder gleich 200 μm vermahlt.

3. Verfahren nach Anspruch 1, dadurch gekennzeichnet, daß man chargenweise arbeitet, daß man das Johannisbrot trocken zu Teilchen mit einem Durchmesser von 0,8 bis 5 mm vermahlt, daß man die Teilchen in Gegenwart von Wasser mit einer Temperatur von 60 bis 98 °C beläßt, daß man die feste Phase sammelt, daß man dieselbe in einem Lufttrockner und gegebenenfalls bei einem unter Atmosphärendruck liegenden Druck bei einer Lufttemperatur von unter 80 °C trocknet, und daß man den Rückstand zu Teilchen mit einem Durchmesser von kleiner als oder gleich 200 μm vermahlt.

4. Verfahren nach Anspruch 1, dadurch gekennzeichnet, daß man den Rückstand durch Lyophilisieren trocknet, daß man denselben granuliert, daß man die Körnchen zerstößt, und daß man den Hauptanteil der Feinteilchen durch einen Heißluftstrom entfernt.

5. Verfahren nach Anspruch 1, dadurch gekennzeichnet, daß das Johannisbrotmehl 30 bis 90 Gew.-% an natürlichen, als Gesamtpolyphenole ausgedrückten Tanninen enthält.

6. Verfahren zur Herstellung eines diätetischen Produktes in einer für die orale Verabreichung geeigneten Form, dadurch gekennzeichnet, daß man das Johannisbrotmehl auf naßem oder trockenem Wege mit Nahrungsmittelzusätzen vermischt, und daß man die Mischung gegebenenfalls in ein Granulat oder zu Tabletten umwandelt.

7. Verfahren zur Herstellung eines diätetischen Produktes in einer für die orale Verabreichung geeigneten Form, dadurch gekennzeichnet, daß man das Johannisbrotmehl einem Getränk oder einer Nachspeise einverleibt.

8. Verfahren nach einem der Ansprüche 6 und 7, dadurch gekennzeichnet, daß man dem diätetischen Produkt Rehydratisierungssalze einverleibt.